# EUROPEAN PATENT APPLICATION

(11) **EP 1 683 528 A1**
(43) Date of publication of application: **26.07.2006**
(21) Application number: 04799708.5
(22) Date of filing: 10.11.2004
(51) Int. Cl.: A61K 47/36, G02C 13/00

(54) **POLYSACCHARIDE-CONTAINING COMPOSITION AND TEAR FILM STABILIZING OPHTHALMIC SOLUTION**

(30) Priority: 10.11.2003 JP 2003380194
(71) Applicant: TORAY INDUSTRIES, INC., Tokyo 103-8666 (JP)
(72) Inventor: SUGIHARA, Y., c/o SANTEN PHARMACEUTICAL CO., LTD, Osaka-shi, Osaka 533-8651 (JP); KIMURA, Akio, c/o SANTEN PHARMACEUTICAL CO., LTD, Osaka-shi, Osaka 533-8651 (JP); NAKAMURA, M., c/o SANTEN PHARMACEUTICAL CO., LTD, Ikoma-shi, Nara 630-0101 (JP); TANAHASHI, Kazuhiro, Otsu-shi, Shiga 5200801 (JP); ARAKI, Miho, Otsu-shi, Shiga 5202143 (JP); TANIGUCHI, Takashi, Yasu-shi, Shiga 5202324 (JP)
(74) Representative: Hart Davis, Jason
(86) International application number: PCT/JP2004/017031
(87) International publication number: WO 2005/046728

(57) **Abstract**

An object of the present invention is to provide a composition which is excellent in safety and capable of dispersing uniformly on the mucous tissue when topically administered to mammals, and an ophthalmic soulution comprising the composition and capable of stabilizing the tear film and keeping the rear film on the eyeball surface snooth. In a composition comprising a polysaccharide, particularly agar, and water as essential components, an ophthalmic solution comprising the agar-containing composition of which an amount of precipitated agar after performing centrifugal separation at with 40,000 xg is less than 65 wt% of a total agar content has an excellent tear film stabilizing effect which was proved by a test on changes of corneal surface irregularity. Therefore, the agar-containing ophthalnic solution of the present invention has the effect of stabilizing the tear film on the eyeball surface over a prolonged period of time and is usable as an artificial tear fluid.

## Description

### Technical Field

The present invention relates to a polysaccharide-containing composition and a tear film stabilizing ophthalmic solution containing the composition.

### Background Art

Various gelling agents and thickeners comprising polysaccharides etc. are used in the fields of drugs, foods, cosmetics, toiletry products, and the like, and various products in the state of a gel and a sol are available on the market. In these fields, technologies for imparting brand-new texture, coating properties, biocompatibility, drug retention, and pharmacological effect by properly controlling the sol/gel state have been demanded.

Among the polysaccharides, agar has already been used widely for foods and so on and is highly safe as is apparent from its entry in the Japanese pharmacopoeia. Also, it is known that agar is useful as a water retaining agent for foods, drugs, cosmetics, and the like because of its water retention effect of suppressing evaporation of moisture. Recently, comparison of water retention effects of various brands of agars has been conducted (In Vitro Cell. Dev. Biol. Plant, 35 94-101 (1999)).

Agar is a sort of polysaccharide contained in the cell wall matrix of various red algae such as Gelidiales and Gracilariaceae, which is obtained by extraction using hot water. Agar is not a uniform substance and typically consists of agarose which does not contain any sulfuric acid group and agaropectin which contains the sulfuric acid group and the like. A proportion of agarose varies depending on the type of the red alga, and the proportion of agarose, for example, in Gelidiales is about 70%.

A low viscosity polysaccharide composition for improving coating properties and drug retention has been known, and a low viscosity liquid polysaccharide composition comprising a microgel and a method for preparing the composition are disclosed in JP-A-2003-128588 and European Patent No. 355908 for example, wherein the composition is prepared by dissolving agar into a water-based solvent at a temperature higher than a gelling temperature of the agar and then cooling the solution to the gelling temperature or below with shearing.

An effect of improving drug delivery to the tissue is disclosed as an application of the polysaccharide composition to the ophthalmology in JP-A-2003-128588, and, therefore, the polysaccharide composition has been known as a useful base for ophthalmic solutions.

Since work operation using computers and the like has become an every day affair, a high incidence of dry eye symptom has become an issue in the ophthalmology. The tear film covering a surface of an eyeball is remarkably thin and smooth, and, when the tear film becomes unstable, the surface looses its smoothness to result in generation of a dried part, which is called a dry spot, in a short time between blinks. The dry spot can sometimes cause exposure of a part of the cornea. Thus, the unstable tear film on the eyeball surface entails feelings of dryness and discomfort of the eye, and frequent exposure of the cornea can cause a serious disorder in the outer ocular area including the cornea and the conjunctiva. From this point of view, the invention disclosed in JP-A- 2001-247466 relates to a system for stabilizing the tear film by neutralizing ion charge by making polyvinylpyrrolidone to be adsorbed to an ionic contact lens and is suitable for an ophthalmic solution for contact lens and a contact lens-wearing solution.

As technologies for applying agar to the ophthalmology, JP-A-2003-128588 and European Patent No. 267015 disclose inventions relating to an ophthalmic solution wherein agar is used as a base of the ophthalmic solution for improving intraocular drug delivery.

Very little has been known about the pharmacological effect of polysaccharides, particularly of agar, and an idea of using agar for stabilizing the tear film on the eyeball surface has not been reported. Particularly, technology of stabilizing a tissue by controlling a sol/gel ratio of a polysaccharide composition comprising agar and a water-based solvent and bringing the tissue into contact with the composition has not been known.

### Disclosure of the Invention

There have been demands for discovery and development of a composition which is excellent in safety and capable of dispersing uniformly on the mucous tissue when topically administered to mammals as well as of improving stability of the tissue brought into contact with the composition. Particularly, in the ophthalmology, there has been a demand for development of an ophthalmic solution containing a composition capable of stabilizing the tear film over a prolonged period of time and keeping the tear film on the eyeball surface smooth.

The inventors have made studies intensively on various compounds and have surprisingly found that, among compositions comprising a polysaccharide such as agar, which is widely used in food or the like, easily available and excellent in safety for human body, and water as essential components, a polysaccharide-containing composition characterized in that an amount of precipitated polysaccharide after performing centrifugal separation with a centrifugal separator at 25°C with 40,000 xg for one hour is less than 65 wt% of a total polysaccharide content has an effect of stabilizing a tissue which is in contact with the composition. In order to further study the stabilization effect, a corneal surface irregularity change test of measuring changes with time in spherical irregularity of the eyeball surface after administering an ophthalmic solution containing a low viscosity agar-containing composition (the spherical irregularity is increased with an increase in irregularity of the shape of the tear film on the corneal surface) was conducted to find that the ophthalmic solution containing agar prominently stabilizes the tear film on the eyeball surface.

More specifically, the present invention relates to:
(1) A polysaccharide-containing composition comprising polysaccharide and water as essential components, characterized in that an amount of precipitated polysaccharide after performing centrifugal separation at 25°C with 40,000 xg for one hour is less than 65 wt% of a total polysaccharide content;
(2) The polysaccharide-containing composition according to the above (1), wherein the amount of precipitated polysaccharide is less than 55 wt%;
(3) The polysacharide-containing composition according to the above (1), wherein the amount of precipitated polysaccharide is less than 30 wt%;
(4) The polysaccharide-containing composition according to any one of the above (1) to (3), wherein the polysaccharide has a concentration of from 0.0001 to 1 wt%;
(5) The polysaccharide-containing composition according to any one of the above (1) to (3), wherein the polysaccharide has a concentration of from 0.0002 to 0.5 wt%;
(6) The polysaccharide-containing composition according to any one of the above (1) to (5), characterized by uniformly dispersing on a mucous membrane when topically administered to a mammal;
(7) The polysaccharide-containing composition according to the above (6), wherein the mucous membrane is an ocular mucous membrane;
(8) The polysaccharide-containing composition according to any one of the above (1) to (7), wherein the polysaccharide is agar;
(9) A method for preparing a polysaccharide-containing composition which is obtainable by heating a composition comprising a polysaccharide and a water-based medium to a gelling temperature of the polysaccharide or higher to dissolve the polysaccharide and then cooling the composition to the gelling temperature or below with applying a shear force, characterized in that an amount of precipitation of the thus-obtained composition after subjecting the composition to centrifugal separation at 25°C with 40,000 xg for one hour is less than 65 wt% of a total polysaccharide content;
(10) A method for preparing a polysaccharide-containing composition comprising a supernatant liquid obtainable by: heating a composition comprising a polysaccharide and a water-based medium to a gelling temperature of the polysaccharide or higher to dissolve the polysaccharide; cooling the composition to the gelling temperature or below with applying a shear force; and performing centrifugal separation at 25°C with 40,000 xg for one hour, characterized in that an amount of precipitation of the thus-obtained composition after subjecting the composition to centrifugal separation with 40,000 xg is less than 65 wt% of a total polysaccharide content;
(11) The method for preparing a polysaccharide-containing composition according to the above (9) or (10), wherein the amount of polysaccharide precipitation is less than 55 wt%;
(12) The method for preparing a polysaccharide-containing composition according to the above (9) or (10), wherein the amount of polysaccharide precipitation is less than 30 wt%;
(13) The method for preparing a polysaccharide-containing composition according to any one of the above (9) to (12), wherein the polysaccharide in the supernatant liquid obtained by the centrifugal separation has a concentration of from 0.0001 to 1 wt%;
(14) The method for preparing a polysaccharide-containing composition according to any one of the above (9) to (12), wherein the polysaccharide in the supernatant liquid obtained by the centrifugal separation has a concentration of from 0.0002 to 0.5 wt%;
(15) A method for preparing a polysaccharide-containing composition comprising heating a composition comprising a polysaccharide and a water-based medium to a gelling temperature of the polysaccharide or higher to dissolve the polysaccharide and cooling the composition to the gelling temperature or below with applying a shear force, characterized in that the thus-obtained composition is diluted to 0.0001 to 1 wt% so that the diluted composition may disperse uniformly on a mucous tissue when topically administered to a mammal;
(16) The method for preparing a polysaccharide-containing composition according to any one of the above (9) to (15), characterized in that the polysaccharide-containing composition is uniformly dispersed on a mucous tissue when topically administered to a mammal;
(17) The method for preparing a polysaccharide-containing composition according to the above (16), wherein the mucous membrane is an ocular mucous membrane;
(18) The method for preparing a polysaccharide-containing composition according to any one of the above (9) to (17), wherein the polysaccharide is agar;
(19) A contact lens-wearing solution or a contact lens preservative solution comprising the polysaccharide-containing composition defined in any one of the above (1) to (8) as at least one of components;
(20) An agar-containing ophthalmic solution characterized by stabilizing a tear film on an eyeball surface;
(21) An agar-containing ophthalmic solution to be used for treating or preventing dry eye by stabilizing a tear film of an eyeball surface;
(22) The agar-containing ophthalmic solution according to the above (20) or (21), comprising an agar composition comprising agar and water as essential components, the agar composition being characterized in that an amount of precipitated agar after performing centrifugal separation at 25°C with 40,000 xg for one hour is less than 65 wt% of a total agar content and being characterized by uniformly dispersing on an ocular surface when administered in the eye;
(23) The agar-containing ophthalmic solution according to any one of the above (20) to (22), wherein a content of the agar is from 0.0001 to 1 wt%;
(24) The agar-containing ophthalmic solution according to any one of the above (20) to (22), wherein a content of the agar is from 0.001 to 0.5 wt%;
(25) The agar-containing ophthalmic solution according to any one of the above (20) to (22), wherein the agar has a weight average molecular weight of from 10,000 to 1,000,000;
(26) The agar-containing ophthalmic solution according to any one of the above (20) to (22), wherein a viscosity of the ophthalmic solution measured with an E type viscometer (at 25°C and a shear rate of 100 s⁻¹) is 30 mPas or lower; and
(27) A system for stabilizing a tear film on an eyeball surface by administering an ophthalmic solution comprising agar.

Polysaccharides usable in the present invention are, in the broadest sense, carbohydrates each of which generates two or more molecules of monosaccharide through hydrolysis, including oligosaccharides such as disaccharide, trisaccharide, tetrasaccharide and the like, and examples thereof are natural polysaccharides, those obtained by processing the natural polysaccharides, synthetic polysaccharides, and so forth. Specific examples of the polysaccharides are agar, agarose, agaropectin, starch, amylose, amilopectin, isolichenan, laminaran, lichenan, glucan, inulin, levan, fructan, galactan, mannan, xylan, arabinan, pentozan, alginic acid, pectinic acid, protuberic acid, chitin, colominic acid, porphyran, fucoidan, ascophyllan, carageenan, pectin, locust bean gum, guar gum, tamarind gum, tara gum, arabic gum, gellan gum, and the like. Among the above, those obtained from seaweeds, such as agar, agarose, agaropectin, laminaran, fructan, galactan, pentozan, alginic acid, chitin, porphyran, fucoidan, ascophyllan, carageenan, and the like are preferable, and, agar, agarose, and agaropectin are more preferable. Particularly agar is preferable.

The agar to be used in the present invention is not particularly limited, and the agar obtainable easily from seaweeds such as Gelidiales may be used in the present invention. Commercially available agars generally contain from 20% to 30% of water, and, in the case of using such agars for the polysaccharide-containing composition of the present invention, the commercially available agar may be used as it is or after physical or chemical modification. Examples of such agar include UP-6, UP-16, UP-37, M-7, M-9, AX-30, AX-100, AX-200, BX-30, BX-100, BX-200, PS-5, PS-6, PS-7, and PS-8, all of which are manufactured by Ina Food Industry Co., Ltd., and the like. The agar to be used in the present invention may be used, in various grades, alone or in combination of two or more.

A content of the polysaccharide in the polysaccharide-containing composition of the present invention is not particularly limited. In the case of using agar as the polysaccharide, a content of agar may preferably be from 0.0001 to 1 wt% without particular limitation thereto. The agar content may more preferably be from 0. 001 to 0.5 wt%, and a yet more preferably from 0.005 to 0.1 wt%. The effect on stabilizing mucous membrane surface is not exhibited sufficiently when the agar content is less than 0.0001 wt%, and, when the content exceeds 1 wt%, a viscosity of the agar-containing composition is raised to deteriorate spread on and penetration into the surface, thereby impairing the feel in instillation in the case of ophthalmic use.

A molecular weight of the polysaccharide to be contained in the polysaccharide-containing composition of the present invention is not particularly limited. Though a molecular weight of agar is not particularly limited, a weight average molecular weight of agar may preferably be from 10,000 to 1,000,000, more preferably from 20,000 to 300,000. It is difficult to maintain the low viscosity of the agar-containing composition when the weight average molecular weight of agar exceeds 1,000,000. The weight average molecular weight of agar can be measured by using the gel permeation chromatography.

An amount of precipitated polysaccharide after performing centrifugal separation of the polysaccharide composition of the present invention by using a centrifugal separator at 25°C with 40,000 xg for one hour may preferably be as small as possible. When the polysaccharide precipitation amount is too large, spread on and penetration into the mucous surface, are deteriorated, and, particularly, feel in instillation is deteriorated when the composition is used for an ophthalmic solution. The polysaccharide precipitation amount may preferably be less than 65 wt%, more preferably less than 55 wt%, and most preferably less than 30 wt%.

In the case of the ophthalmic use, a viscosity of the ophthalmic solution may preferably be adjusted to be 30 mPas (30 cPs) or less as measured with an E type viscometer (25°C, shear rate: 100 s⁻¹). A more preferable viscosity of the ophthalmic solution is 10 mPas or less. The feel in instillation tends to be deteriorated when the viscosity of the ophthalmic solution exceeds 30 mPas.

The polysaccharide-containing composition of the present invention may preferably include a water-based medium as one of components. The water-based medium is a liquid substance containing water as the main component, and components other than the water are not particularly limited. The water content may preferably exceed 80 wt%, more preferably exceed 90 wt%.

In the case of using agar in the polysaccharide-containing composition of the present invention, a form of agar is not particularly limited insofar as an amount of precipitated agar after centrifugal separation with a centrifugal separator at 25°C with 40,000 xg for one hour is less than 65 wt% of a total agar content. For example, it is possible to use agar in a perfectly dissolved state, partly dissolved state, or in a state where a part thereof is dispersed as agar particles. The state wherein a part of agar is dispersed as agar particles means that particulate agar is dispersed into water in addition to the dissolved agar, and a particle diameter of the particulate agar may preferably be 100 µm or less. The particle diameter may more preferably be 20 µm or less yet more preferably 10 µm or less. When the particle diameter of agar exceeds 100 µm, a preservation stability of the ophthalmic solution is deteriorated, and the ophthalmic solution can be deteriorated in feel in instillation due to foreign matter sensation when instilled. A form of the particulate agar is not particularly limited, and examples of the form include a spherical form, an oval form, and other atypical forms.

The polysaccharide-containing composition of the present invention is obtainable by heating a composition comprising the polysaccharide and the water-based medium to a gelling temperature or more, preferably to a temperature higher than the gelling temperature by 20°C, to give a transparent and uniform solution and then cooling the composition to a temperature lower than the gelling temperature by 20°C under applying a stress. This method is most preferred in view of its advantage of giving the uniform composition.

Though it is possible to obtain the agar-containing ophthalmic solution of the present invention by dissolving or dispersing agar into water, it is possible to reduce the viscosity of the agar-containing ophthalmic solution by heating an aqueous solution containing agar to give the transparent and uniform solution and then cooling the solution under applying stress or performing ultrasonic irradiation and the like as required.

Though any of vibration, stirring, compression, pulverization, and the like may be performed as the method of applying the stress, the stirring is the most preferable because a shear force is applied to the liquid by the stirring. A device such as a magnetic stirrer, a mechanical stirrer, a mixer, a shaker, a rotor, and a homogenizer may be used for the stirring. Examples of cooling means include air-cooling, water-cooling, icecooling, solvent-cooling, wind-cooling, and the like. Cooling to the gel transition temperature or below is sufficient in principle; however, from the practical point of view, the composition is cooled to a temperature lower than the gel transition temperature by 20°C, or to about 20°C since the polysaccharide-containing composition of the invention is usually used at a room temperature or below. It is possible to obtain the agar-containing composition of the present invention also by collecting a supernatant obtainable by performing centrifugal separation with a centrifugal separator at 25°C with 40,000 xg for one hour after the cooling. Also, the agar-containing composition is obtainable by adjusting a concentration of the composition obtained after the cooling to 0.0001 to 1 wt% through dilution with the use of the water-based medium.

Examples of a preferable mode of the agar-containing ophthalmic solution of the present invention include an ophthalmic solution containing from 0.0001 to 1 wt% of agar having a weight average molecular weight of 10,000 to 1,000,000 and having a viscosity of 30 mPas or less, and a more preferable mode is an ophthalmic solution containing from 0.001 to 0.5 wt% of agar having a weight average molecular weight of 20,000 to 300,000 and having a viscosity of 10 mPas or less.

It is possible to prepare an ophthalmic solution, an ointment, and the like by adding to the polysaccharide-containing composition, particularly to the agar-containing composition, of the present invention an isotonic agent, a buffer, a pH adjustor, a solubilizing agent, a stabilizer, or a preservative. An object of the polysaccharide-containing composition and the ophthalmic solution of the present invention is the stabilization of the tissue, particularly of the tear film, brought into contact with the composition or the ophthalmic solution, and a drug can be added to the polysaccharide-containing composition and the ophthalmic solution insofar as the medical substance does not impair the object.

Examples of the drug include an antibacterial 1 agent, an anti-inflammatory agent, an antihistaminic agent, an antiglaucomic agent, an antiallergic agent, an immunosuppressor, an antimetabolic agent, and the like.

Examples of the isotonic agent include glycerin, propyleneglycol, sodium chloride, potassium chloride, sorbitol, mannitol, and the like.

Examples of the buffer include phosphoric acid, phosphoric acid salt, citric acid, acetic acid, ε-aminocaproic acid, trometamol, and the like.

Examples of the pH adjustor include hydrochloric acid, citric acid, phosphoric acid, acetic acid, sodium hydroxide, potassium hydroxide, boric acid, borax, sodium carbonate, sodium hydrogencarbonate, and the like.

Examples of the solubilizing agent to be added when the drug or the other additive is hardly soluble to water include polysorbate 80, polyoxyethylene hydrogenated castor oil 60, macrogol 4000, and the like.

Examples of the stabilizer include edetic acid, sodium edetate, and the like.

Examples of the preservative include sorbic acid, potassium sorbate, benzalkonium chloride, benzethonium chloride, methyl parahydroxybenzoate, propyl parahydroxybenzoate, chlorobutanol, and the like, and these preservatives may be used in combination.

In the case of formulating the polysaccharide-containing composition, particularly the agar-containing composition, of the present invention, to pharmaceutical preparations, a pH may preferably be set to 4.0 to 8.0, and an osmotic pressure may preferably be set to about 1.0.

Examples of preferable mode of use of the composition of the present invention include a hard or soft contact lens-wearing solution or a contact lens preservative solution, and the composition is particularly suitable for patients who suffers from the dry eye and have difficulty in wearing contact lenses.

Though the frequency of administration may be appropriately selected depending on symptom, age, dosage form, and the like, it is sufficient that the agar-containing composition of the present invention is administered once per day.

As is apparent from the temporal measurements of the spherical irregularity on the eyeball surface after the instillation of the ophthalmic solution comprising the agar-containing composition of the present invention, which will be described in detail later in the section of "Test on Changes of Corneal Surface Irregularity", the agar-containing ophthalmic solution of the present invention has an effect of stabilizing the tear film on the eyeball surface over a prolonged period of time. Also, the ophthalmic solution of the present invention acts as an artificial tear fluid in the case where lacrimal secretion is insufficient. Further, since the agar-containing composition of the present invention exhibits the tear fluid stabilizing effect with a small content of agar, in the case of the ophthalmic use of the agar-containing composition, the composition enables a low viscosity of the ophthalmic solution, good feel in instillation, and feel of moistness and is excellent in safety. In addition, as is apparent from comparison between agar and trehalose, which retains moisture as agar, agar not only retains moisture but also stabilizes the tear fluid due to its specific properties that are not observed with trehalose.

That is to say, the inventors have found that the agar itself has the tear fluid stabilizing effect. In order to exhibit the tear fluid stabilizing effect, it is necessary to disperse agar uniformly on the mucous membrane surface. The mechanism of the uniform dispersion is not yet clarified but can be considered as follows. That is, when agar is in the gel state, a hydrogen bonding is formed between molecular chains of agar molecules so that the agar molecules incorporate water molecules to establish a helical structure, thereby achieving a higher-order and stronger structure and being precipitated by centrifugation. In turn, some of the agar molecules existing in the water-based medium are highly free and hydrous without forming the stable helical structure, and such agar molecules are not precipitated by centrifugation. When a liquid containing such agar molecules is administered on the ocular mucous membrane surface, the agar molecules spread widely on the ocular mucous membrane surface to exhibit the tear fluid stabilizing effect. It is considered that administration of a gel formed from the same amount of agar and having the high order structure cannot achieve the tear fluid stabilization since the gel is reduced in contact area on the ocular mucous membrane surface and does not spread when it is in contact with the ocular mucous membrane surface. It is considered that the same applies to the other mucous membranes than the ocular mucous membrane.

### Brief Description of the Drawing

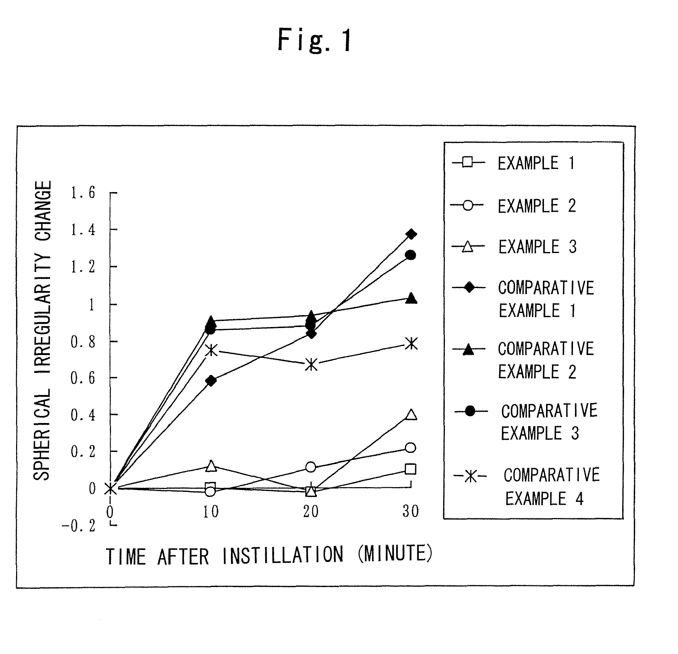

Fig. 1 is a graph showing time shift of spherical irregularity changes.

### Best Mode for Carrying out the Invention

The present invention will hereinafter be described through embodiments, but it should be understood that the embodiments are given only by way of example and not for the purpose of limiting the scope of the present invention.

### [Test on Changes of Corneal Surface Irregularity]

In this test, corneal surface irregularities (irregularities of the tear film) after instilling test ophthalmic solutions were measured with a corneal shape measurement device for the purpose of evaluating a tear fluid stabilizing effect of each of the test ophthalmic solutions.

### (1) Preparation of Test Solutions

### Example 1

2.6 g of concentrated glycerin was placed into a 100 ml flask as an isotonic agent, and then purified water was added until the volume reached to 100 ml. To this solution, 0.1 g of agar (AX-30; weight average molecular weight: about 90,000) was added, followed by heating it to about 100°C to dissolve the agar and then by cooling it to the room temperature with stirring with a magnetic stirrer, thereby obtaining a test solution 1 (viscosity: 2.9 mPas) which was slightly white.

### Example 2

A transparent test solution 2 (viscosity: 1.1 mPas) was obtained by performing the same operation as Example 1 except for adding 0.01 g of the agar (AX-30) instead of 0.1 g of agar (AX-30).

### Example 3

0.5 g of agar (UP-6; weight average molecular weight: about 220,000) was added to 100 ml of purified water, followed by heating it to about 100°C to dissolve the agar and then by cooling it to the room temperature with stirring with a magnetic stirrer, thereby obtaining a white agar solution. 2.6 g of concentrated glycerin was added to 20 ml of the agar solution, followed by adding thereto purified water until the volume reached to 100 ml, thereby obtaining a white test solution 3 (viscosity: 1.5 mPas).

### Comparative Example 1

2.6 g of concentrated glycerin was placed into a 100 ml flask as an isotonic agent, and then purified water was added until the volume reached to 100 ml, thereby obtaining a transparent comparative test solution 1 (viscosity: 1.0 mPas).

### Comparative Example 2

0.9 g of sodium chloride was placed into a 100 mal measuring flask as an isotonic agent, and then 1.0 g of trehalose was added, followed by dissolving it with stirring, thereby obtaining a transparent comparative test solution 2 (viscosity: 1.0 mPas).

### Comparative Example 3

0.5 g of agar (AX-30) was added to 100 ml of purified water, followed by heating it to about 100°C to dissolve the agar and then by cooling it to the room temperature with stirring with a homomixer, thereby obtaining a white agar solution. 2.6 g of concentrated glycerin was added to the agar solution to obtain a white comparative test solution 3 (viscosity: about 20 mPas).

### Comparative Example 4

0.5 g of agar (UP-6) was added to 100 ml of purified water, followed by heating it to about 100°C to dissolve the agar and then by cooling it to the room temperature with stirring with a magnetic stirrer, thereby obtaining a white agar solution. 2.6 g of concentrated glycerin was added to the agar solution to obtain a white comparative test solution 4 (viscosity: 38.1 mPas) .

### (2) Measurement of Amount of Precipitated Agar

3 mL each of the test solutions obtained by the above preparation methods were placed into a safety oven (SPH-101; manufactured by Tabai Espec Corp.) at 60°C and left for 2 hours. After that, the temperature of the oven was raised to 120°C which was kept for 2 hours to remove moisture. Then, after cooling it to 25°C in a desiccator, measurements of amounts of precipitations were conducted with "AE 160" (manufactured by Mettler-Toledo International Inc.). From a volume of each test solution and a precipitated agar amount obtained therefrom, a total agar content in 10 mL of the test solution was calculated. Further, 10 mL each of the test solutions were subjected to centrifugal separation with an inverter multipurpose high-speed cooling centrifuge (6930; manufactured by Kubota Corporation; rotor: RA-120) at 25°C with 40,000 xg for one hour. Then, the obtained precipitations were subjected to the heating at 60°C for 2 hours and then 120°C for 2 hours in the same manner as described above to remove moisture, and then precipitated agar amounts were measured after cooling. A ratio of the precipitated agar amount to the total agar amount in each of the test solutions was calculated. In order to remove glycerin, the dried samples were washed with acetone and then dried before the weight measurement.

### (3) Administration Method and Measurement Method

Each 20 µl of the test solutions obtained by the above preparation methods were instilled to male Japanese white rabbits under general anesthesia, followed by measuring of the corneal surface shapes at 0 (immediately after the instillation), 10, 20, and 30 minutes after the instillation with the eyelids forcibly retracted, with a corneal shape measurement device (TMS-2N; manufactured by Tomey Corporation) to calculate spherical irregularities (spherical irregularity is increased with an increase in irregularity of the shape of the tear film on the corneal surface) and spherical irregularity changes (a spherical irregularly change means a value obtained by subtracting the spherical irregularity immediately after the instillation from the temporal spherical irregularity). The results are shown in Table 1, and time shift of the spherical irregularity changes is shown in Fig. 1. Note that the spherical irregularity of each of the test solutions is an average value of the four or five values.

**Table 1**

| | Sample | Component (Content) | Viscosity (mPas) | Spherical Irregularity Change after 30 Minutes | Ratio of Precipitated Agar (%) |
|---|---|---|---|---|---|
| Ex. 1 | Test Solution 1 | AX-30 (0.1%) | 2.9 | 0.10 | 22.2 |
| Ex. 2 | Test Solution 2 | AX-30 (0.01%) | 1.1 | 0.22 | ≤0.1 |
| Ex. 3 | Test Solution 3 | UP-6 (0.1%) | 1.5 | 0.40 | 21.5 |
| Comp. Ex. 1 | Comparative Test Solution 1 | - | 1.2 | 1.37 | - |
| Comp. Ex. 2 | Comparative Test Solution 2 | Trehalose (1%) | 0.9 | 1.03 | - |
| Comp. Ex. 3 | Comparative Test Solution 3 | AX-30 (0.5%) | | 1.26 | 67.2 |
| Comp. Ex. 4 | Comparative Test Solution 4 | UP-6 (0.5%) | 38.1 | 0.78 | 74.8 |

### (4) Analysis

As is apparent from Table 1 and Fig. 1, the agar-containing solutions of Examples 1 to 3 have the tear film stabilizing effect which is far more excellent than that of Comparative Example 1 containing not agar but only the base. Comparative Example 2 containing trehalose having the water retention property exhibits the tear film stabilizing effect which is almost the same as that of Comparative Example 1. Since the significant stabilizing effect is observed with Examples 1 to 3 each of which contains agar, it is clarified that the tear film stabilizing effect is the pharmacological effect of agar resulting from the uniform dispersion of agar on the mucous surface and is not attributable to the water retention property.

### Industrial Applicability

The present invention provides the composition which is excellent in safety and capable of uniformly dispersing on the mucous tissue when topically administered to mammals. The composition improves stability of the tissue brought into contact with the composition and, particularly, in the ophthalmology, stabilizes the tear film over a prolonged period of time to keep the tear film on the eyeball surface smooth.

## Claims

1. A polysaccharide-containing composition comprising polysaccharide and water as essential components, **characterized in that** an amount of precipitated polysaccharide after performing centrifugal separation at 25°C with 40,000 xg for one hour is less than 65 wt% of a total polysaccharide content.

2. The polysaccharide-containing composition as claimed in claim 1, wherein the amount of precipitated polysaccharide is less than 55 wt%.

3. The polysaccharide-containing composition as claimed in claim 1, wherein the amount of precipitated polysaccharide is less than 30 wt%.

4. The polysaccharide-containing composition as claimed in any one of claims 1 to 3, wherein the polysaccharide has a concentration of from 0.0001 to 1 wt%.

5. The polysaccharide-containing composition as claimed in any one of claims 1 to 3, wherein the polysaccharide has a concentration of from 0.0002 to 0.5 wt%.

6. The polysaccharide-containing composition as claimed in any one of claims 1 to 5, **characterized by** uniformly dispersing on a mucous membrane when topically administered to a mammal.

7. The polysaccharide-containing composition as claimed in claim 6, wherein the mucous membrane is an ocular mucous membrane.

8. The polysaccharide-containing composition as claimed in any one of claims 1 to 7, wherein the polysaccharide is agar.

9. A method for preparing a polysaccharide-containing composition which is obtainable by heating a composition comprising a polysaccharide and a water-based medium to a gelling temperature of the polysaccharide or higher to dissolve the polysaccharide and then cooling the composition to the gelling temperature or below with applying a shear force, **characterized in that** an amount of precipitation of the thus-obtained composition after subjecting the composition to centrifugal separation at 25°C with 40,000 xg for one hour is less than 65 wt% of a total polysaccharide content.

10. A method for preparing a polysaccharide-containing composition comprising a supernatant liquid obtainable by: heating a composition comprising a polysaccharide and a water-based medium to a gelling temperature of the polysaccharide or higher to dissolve the polysaccharide; cooling the composition to the gelling temperature or below with applying a shear force; and performing centrifugal separation at 25°C with 40,000 xg for one hour, characterizes in that an amount of precipitation of the thus-obtained composition after subjecting the composition to centrifugal separation with 40,000 xg is less than 65 wt% of a total polysaccharide content.

11. The method for preparing a polysaccharide-containing composition as claimed in claim 9 or 10, wherein the amount of polysaccharide precipitation is less than 55 wt%.

12. The method for preparing a polysaccharide-containing composition as claimed in claim 9 or 10, wherein the amount of polysaccharide precipitation is less than 30 wt%

13. The method for preparing a polysaccharide-containing composition as claimed in any one of claims 9 to 12, wherein the polysaccharide in the supernatant liquid obtained by the centrifugal separation has a concentration of from 0.0001 to 1 wt%.

14. The method for preparing a polysaccharide-containing composition as claimed in any one of claims 9 to 12, wherein the polysaccharide in the supernatant liquid obtained by the centrifugal separation has a concentration of from 0.0002 to 0.5 wt%.

15. A method for preparing a polysaccharide-containing composition comprising heating a composition comprising a polysaccharide and a water-based medium to a gelling temperature of the polysaccharide or higher to dissolve the polysaccharide and cooling the composition to the gelling temperature or below with applying a shear force, **characterized in that** the thus-obtained composition is diluted to 0.0001 to 1 wt%.

16. The method for preparing a polysaccharide-containing composition as claimed in any one of claims 9 to 15, **characterized in that** the polysaccharide-containing composition is uniformly dispersed on a mucous tissue when topically administered to a mammal.

17. The method for preparing a polysaccharide-containing composition as claimed in claim 16, wherein the mucous membrane is an ocular mucous membrane.

18. The method for preparing a polysaccharide-containing composition as claimed in any one of claims 9 to 17, wherein the polysaccharide is agar.

19. A contact lens-wearing solution or a contact lens preservative solution comprising the polysaccharide-containing composition defined in any one of claims 1 to 8 as at least one of components.

20. An agar-containing ophthalmic solution **characterized by** stabilizing a tear film on an eyeball surface.

21. An agar-containing ophthalmic solution to be used for treating or preventing dry eye by stabilizing a tear film of an eyeball surface.

22. The agar-containing ophthalmic solution as claimed in claim 20 or 21, comprising an agar composition comprising agar and water as essential components, the agar composition being **characterized in that** an amount of precipitated agar after performing centrifugal separation at 25°C with 40,000 xg for one hour is less than 65 wt% of a total agar content and being **characterized by** uniformly dispersing on an ocular surface when administered in the eye.

23. The agar-containing ophthalmic solution as claimed in any one of claims 20 to 22, wherein a content of the agar is from 0.0001 to 1 wt%.

24. The agar-containing ophthalmic solution as claimed in any one of claims 20 to 22, wherein a content of the agar is from 0.001 to 0.5 wt%.

25. The agar-containing ophthalmic solution as claimed in any one of claims 20 to 22, wherein the agar has a weight average molecular weight of from 10,000 to 1,000,000.

26. The agar-containing ophthalmic solution as claimed in any one of claims 20 to 22, wherein a viscosity of the ophthalmic solution measured with an E type viscometer (at 25°C and a shear rate of 100 s⁻¹) is 30 mPas or lower.

27. A system for stabilizing a tear film on an eyeball surface by administering an ophthalmic solution comprising agar.
